# EUROPEAN PATENT SPECIFICATION

(11) **EP 1 792 575 B1**
(45) Date of publication and mention of the grant of the patent: **30.06.2010**
(21) Application number: 06256106.3
(22) Date of filing: 29.11.2006
(51) Int. Cl.: A61B 17/12

(54) **Embolic device delivery system**
Embolievorrichtung Abgabesystem
Sytème de placement d'un dispositif d'embolisation

(30) Priority: 30.11.2005 US 290954
(43) Date of publication of application: 06.06.2007
(73) Proprietor: Codman & Shurtleff, Inc., New Brunswick, NJ 08933 (US)
(72) Inventor: Farnan, Robert C., NJ 07675 (US)
(74) Representative: Gover, Richard Paul

(56) References cited:
- WO-A-93/11825
- WO-A-96/00104
- WO-A2-94/06502

## Description

The present invention is related to the delivery of embolic occlusion devices. Disclosed are occlusion device deployment systems and methods for mechanically deploying occlusion devices at a preselected location within a patient, in an accurate and rapid manner. The deployment systems and methods are particularly suited for deploying an embolic coil at a location of concern within the vasculature of a patient.

The use of catheter delivery systems for positioning and deploying therapeutic devices, such as dilation balloons, stents and embolic coils, in the vasculature of the human body has become a standard procedure for treating endovascular diseases. It has been found that such devices are particularly useful in treating areas where traditional operational procedures are impossible or pose a great risk to the patient, for example in the treatment of aneurysms in cranial blood vessels. Due to the delicate tissue surrounding cranial blood vessels, especially for example brain tissue, it is very difficult and often risky to perform surgical procedures to treat defects of the cranial blood vessels. Advancements in catheter deployment systems have provided an alternative treatment in such cases. Some of the advantages of catheter delivery systems are that they provide methods for treating blood vessels by an approach that has been found to reduce the risk of trauma to the surrounding tissue, and they also allow for treatment of blood vessels that in the past would have been considered inoperable.

Typically, these procedures involve inserting the distal end of a delivery catheter into the vasculature of a patient and guiding it through the vasculature to a predetermined delivery site. A vascular occlusion device, such as an embolic coil, is attached to the end of a delivery member which pushes the coil through the catheter and out of the distal end of the catheter into the delivery site. Some of the problems that have been associated with these procedures relate to the accuracy of coil placement. For example, the force of the coil exiting the delivery catheter may cause the coil to over shoot the predetermined site or dislodge previously deployed coils. Also, once the coil is pushed out of the distal end of the catheter, the coil cannot be retracted and may migrate to an undesired location. Often, retrieving and repositioning the coil requires a separate procedure and has the potential to expose the patient to additional risk.

In response to the above mentioned concerns, numerous devices and release mechanisms have been developed in an attempt to provide a deployment system which allows control of the occlusion device after the device has been delivered by the catheter and to also provide a rapid release or detachment mechanism to release the device once it is in place. One such device, which is disclosed in US-5108407, includes a fibre optic cable including a connector device mounted to the end to the optic fibre. An embolic coil is attached to the connector device by a heat releasable adhesive. Laser light is transmitted through the fibre optic cable to increase the temperature of the connector device, which melts the adhesive and releases the embolic coil. One drawback to using this type of system is the potential risk of melted adhesives contaminating the blood stream.

Another coil deployment system employs a pusher member having an embolic coil attached to the pusher member by a connector fibre which is capable of being broken by heat, is disclosed in US-6478773. The pusher member of this arrangement includes an electrical resistance heating coil through which the connector fibre is passed. Electrical current is supplied to the heating coil by a power source connected to the heating coil via wires extending through an internal lumen of the pusher. The power source is activated to increase the temperature of the heating coil which breaks the connector fibre. One drawback is that connecting the resistance heating coil to the power source requires running multiple wires through the pusher member. Additionally, the electrical current travelling through the wires may create stray electromagnetic fields that have the potential to interfere with other surgical and monitoring equipment.

Yet another embolic coil positioning and delivery system, which is described in US-5989242, includes catheter having a shape memory alloy connector attached to the distal end of the catheter. The connector includes a socket having a pair of spaced-apart fingers which are responsive to a change in temperature. The fingers are bent towards each other and hold a ball which is connected to an end of an embolic coil. The connector absorbs laser light transmitted through an optical cable and transforms the light into heat energy. The heat energy raises the temperature of the connector and opens the fingers, thereby releasing the embolic coil. This type of ball and socket connection is rigid and causes the catheter to be stiff, making it difficult to guide the catheter through the vasculature of the body.

A pusher-vasoocclusive coil assembly is described in WO 93/11825, including a coil having an enlarged member at its proximal end and the pusher has a keyway at its distal end that receives the enlarged member in interlocking engagement. Further, the above-identified delivery systems typically require electronic equipment powered by a power source. If the electronic equipment is defective or the power source fails, the procedure may be prolonged while the equipment is repaired or replaced. Prolonging the procedure may expose the patient to additional risk.

Therefore, a need remains for a rapid release vascular occlusion deployment system or method that can function without electrical equipment or a power supply, does not develop chemical debris, is simple to manufacture, flexible and easy to guide through the vasculature of the body, provides excellent control over the occlusion device, and reduces the possibility of interference with other surgical and/or monitoring equipment.

The present invention embodies deployment systems and methods for accurately and rapidly deploying a vascular occlusion device at a location of concern within the vasculature of a patient. The deployment system can employ an elongated flexible delivery catheter for guiding a deployment unit to a location of concern within a patient. The deployment unit includes a pusher for pushing and guiding the vascular occlusion device, such as an embolic coil, through the delivery catheter to the location of concern.

Preferably, the pusher has a proximal end portion and a distal end portion, and a channel extending between the proximal end portion and the distal end portion. The pusher also includes an elongated member which is slidably located within the channel. The elongate member and the pusher are also able to rotate with respect to one another, i.e., the elongated member is able to rotate within the channel of the pusher, and the pusher is able to rotate around the elongated member.

The distal end portion of the elongated member includes a connecting projection, plug or cam follower extending in a generally radial direction therefrom. An embolic device can be removably secured to the elongated member by engaging the connecting projection or cam follower with a channel or cam pathway defined by a proximal end portion of the embolic device. The connecting projection or cam follower can be engaged with the channel or cam pathway by positioning the elongated member so that the connecting projection or cam follower is at the entrance of the channel or cam pathway. The elongated member is rotated in the direction of the pathway, such as the wind, of the channel so that the connecting projection or cam follower moves within the channel or cam pathway. In one preferred embodiment, the channel or cam pathway is helically configured so that as the connecting projection or cam follower is helically threaded into the channel or cam pathway, the elongated member advances distally with respect to the embolic device and/or the embolic device advances proximally with respect to the elongated member.

The embolic device can be released from the pusher after the device is positioned endoluminally. Prior to release, the distal end portion of the pusher is contacted with the proximal end portion of the embolic device attached to the elongated member so that the embolic device will not rotate independently of the pusher. This imparts an engaged state at which the embolic device is maintained in a substantially stationary position by engagement between the pusher and the embolic device. While in this engaged state, the elongated member is rotated in a direction opposite to the direction in which the elongated member had been rotated in order to secure the embolic device to the elongated member. The connecting projection or cam follower again moves along the channel or cam pathway until release, such as by unthreading the connecting projection or cam follower. The embolic device is thereby released from the deployment system at a desired deployment location.

The device of the invention can be used in method of employing a pusher to deliver an embolic device to a location within the vasculature of a patient, the pusher including an elongated member having a proximal end portion, a distal end portion and a connecting projection along the distal end portion, the connecting projection being engageable with a pathway of an embolic device to removably attach the embolic device to the elongated member, comprising:
manipulating the pusher and guiding the embolic device to a preselected location within the vasculature of a patient;
maintaining the embolic device in a substantially stationary position; and
disengaging the connecting projection by rotating the elongated member, thereby releasing the embolic device from the pusher.

The device of the invention can be used in a method of employing a pusher to deliver an embolic device to a location within the vasculature of a patient, the pusher including an elongated member having a proximal end portion, a distal end portion and a connecting projection along the distal end portion, the connecting projection being threadably engageable with a channel of an embolic device to removably attach the embolic device to the elongated member, comprising:
manipulating the pusher and guiding the embolic device to a preselected location within the vasculature of a patient;
maintaining the elongated member in a substantially stationary position; and
unthreading the connecting projection by rotating the embolic device, thereby releasing the embolic device from the pusher to deploy the embolic device within the vasculature.

Embodiments of the invention will now be described by way of example with reference to the accompanying drawings, in which:
Fig. 1 is an enlarged partially sectioned view of an occlusion device deployment system in accordance with a preferred embodiment of the present invention;
Fig. 2 is an exploded view of the deployment unit illustrated in Fig. 1;
Figs. 3 and 3a are enlarged perspective views of one embodiment of the embolic device headpiece;
Figs. 4 and 4a are enlarged perspective views of one embodiment of the pusher headpiece;
Fig. 5 is an enlarged partially sectioned view of the deployment system of Fig. 1 shown prior to deployment;
Fig. 6 is an enlarged partially sectioned view of the deployment system of Fig. 1 shown after the embolic device has exited the delivery catheter and shown with the distal end portion of the pusher and the embolic device being separated;
Fig. 7 is an enlarged partially sectioned view of the deployment system of Fig. 1 shown with the pusher engaging the embolic device after the embolic device has exited the delivery catheter; and
Fig. 8 is an enlarged partially sectioned view of the deployment system of Fig. 1 shown after the embolic device has been released.

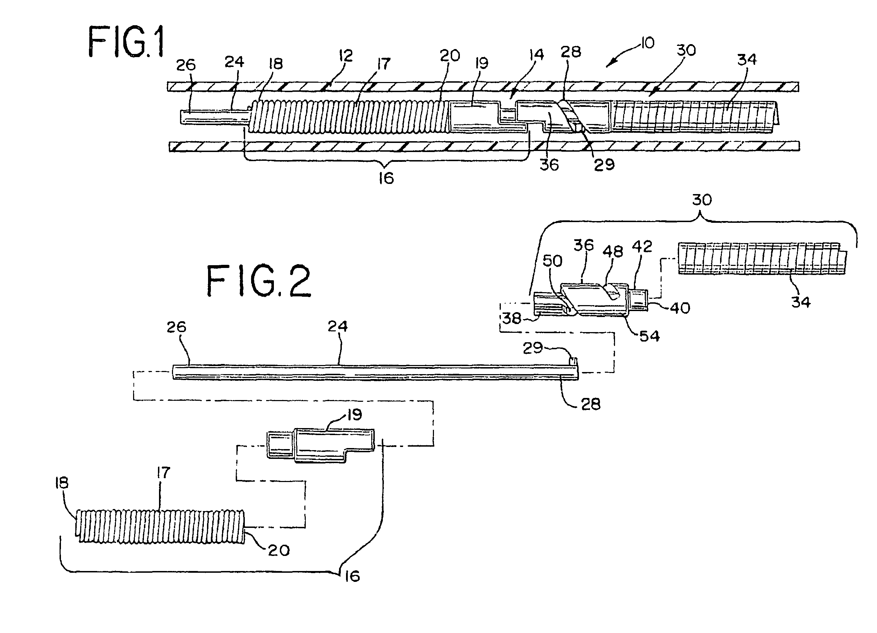

Referring to the drawings, Fig. 1 shows a deployment system 10 which includes an elongated flexible delivery catheter 12 which can be inserted into the vasculature of a patient and used to guide a deployment unit, generally designated at 14, to a preselected site in a manner generally known in the art. One of ordinary skill in the art will appreciate that the delivery catheter 12 and the deployment unit 14 are much longer than illustrated in the figures.

Referring to Figs. 1 and 2, the deployment unit 14 includes an elongated flexible pusher 16 which can be comprised of a delivery tube 17 and a pusher headpiece 19. The delivery tube 17 has a proximal end portion 18 and a distal end portion 20. An internal lumen (not shown) extends from the proximal end portion 18 of the delivery tube 17 through the pusher headpiece 19. The delivery tube 17 can be any suitable type of delivery tube generally known in the art that has sufficient column strength to push an embolic device through a delivery catheter and sufficient flexibility to be guided through tortuous pathways within the vasculature of a patient. Additionally, the delivery tube preferably has the ability to resist torque applied to the delivery tube during release of the embolic device, as describe below. For example, the delivery tube can be comprised a coil wound wire, or the delivery tube can be a flexible polymer sheath.

An elongated member 24 is slidably disposed within the channel of the pusher 16, i.e., the elongated member 24 is relatively moveable in a proximal and a distal direction with respect to the pusher 16. Additionally, the elongated member 24 and the pusher 16 are able to rotate with respect to one another, i.e., the elongated member 24 is able to rotate within the channel of the pusher 16 and the pusher is able to rotate around the elongated member 24.

The elongated member 24 is preferably comprised of a metallic or polymeric material which has tensile and flex properties that allow the elongated member to be easily guided through tortuous paths within the patient.

The elongated member 24 includes a proximal end portion 26 and a distal end portion 28. The distal end portion 28 can be positioned to extend out of the pusher headpiece 19. The elongated member 24 also includes a connecting projection, cam follower or plug 29 extending in a generally radial direction from the distal end portion 28 of the elongated member 24. In the illustrated arrangement, the connecting projection, cam follower or plug 29 is perpendicular to the axis of the elongated member 24. The connecting projection, cam follower or plug 29 can be used to removably secure an embolic device 30 to the elongated member 24, as described in more detail below.

The elongated member 24 and the connecting projection, cam follower or plug 29 can be of a unitary construction. For example the projection, cam follower or plug 29 can be formed as a distal bend of the elongated member 24 which may have a modified characteristic such as an end of a different size. Alternatively, the connecting projection, cam follower or plug 29 can be attached to the elongated member 24 by any suitable attachment method known in the art, such as welding, force fitting, soldering or adhering with adhesive.

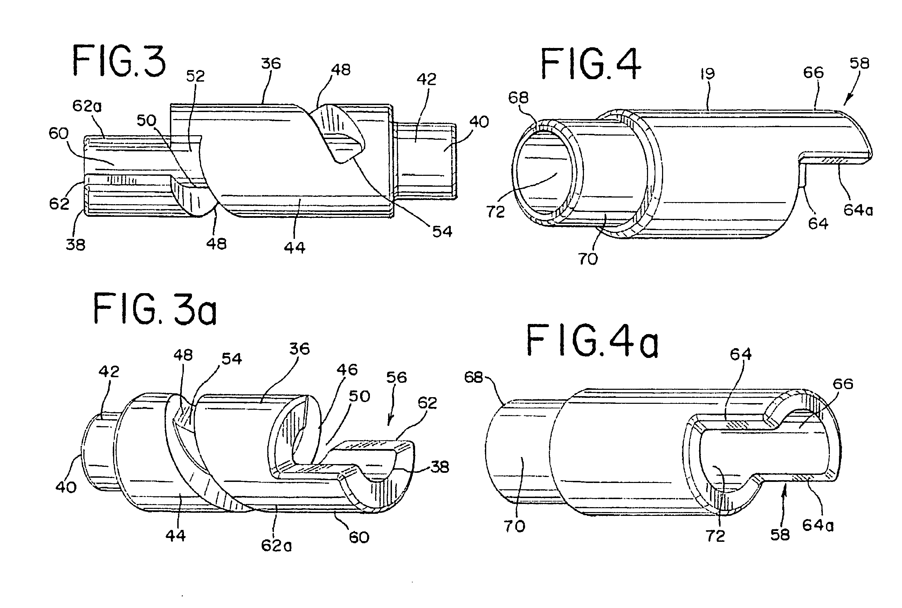

The embolic device 30 is preferably an embolic device assembly that includes an embolic element 34 and a headpiece 36. As illustrated in Figs. 2, 3 and 3a, the embolic device headpiece 36 has a proximal end portion 38 and a distal end portion 40. The distal end portion 40 includes a joining element 42, which is illustratively shown as a cylindrical projection, for connecting the embolic element 34 to the headpiece 36. The embolic element 34 and joining element 42 may be connected by weld, solder, adhesive or any other suitable attachment method known in the art. Illustratively, the embolic device 30 comprises the headpiece 36 and the embolic element 34 which are separate components that are secured together; however, it will be understood by one of ordinary skill in the art that the embolic element 34 and the headpiece 36 can be of a unitary construction to form embolic device 30.

The embolic element 34 is preferably an embolic coil which can be of the type which takes a substantially linear configuration for being advanced through the delivery catheter and a randomly oriented relaxed condition after it is released from the catheter. Alternatively, the embolic element 34 may be any other type of embolic element which may take on various forms and configurations, such as hydrogels, foams, bioactive coils, braids, cables and hybrid devices.

In the illustrated embodiment, the headpiece 36 includes a circumferential wall 44 which defines a cavity 46. The circumferential wall 44 also includes a channel or cam pathway 48 into which the connecting projection, cam follower or plug 29 of the elongated member 24 can be threaded to secure the embolic device 30 to the elongated member 24. Illustratively, the channel or cam pathway 48 extends through the circumferential wall 44. It is also contemplated that the channel or cam pathway 48 could be comprised of a groove located on the inner surface of circumferential wall, but does not extend through the circumferential wall. Additionally, the channel or cam pathway 48 is preferably configured in the illustrated helical configuration; however, it is contemplated that the channel or cam pathway 48 can be of a substantially circular configuration or other turn configuration.

Preferably, including when the channel or cam pathway 48 is of a helical configuration, the channel or cam pathway 48 includes an entrance 50 which can include a notch 52 that is sized to accommodate movement of the connecting projection, cam follower or plug 29 along it. For example, the notch 52 can be slightly larger than the approximate dimensions of the connecting projection, cam follower or plug 29. The notch 52 aids in advancing the connecting projection, cam follower or plug 29 into the channel or cam pathway 48 and withdrawing the connecting projection, cam follower or plug 29 from the channel or cam pathway 48 when the headpiece 36 is engaged with headpiece 19. The channel or cam pathway 48 also includes an end wall 54 which is located distally of the entrance 50.

To connect the embolic device 30 to the elongated member 24, the connecting projection, cam follower or plug 29 is positioned within the notch 52 of the entrance 54 of the channel or cam pathway 48. The elongated member 24 is then rotated to thread the connecting member, cam follower or plug 29 into the channel or cam pathway 48. In the illustrated embodiment, the elongated member 24 is rotated in a clockwise direction to thread the connecting projection, cam follower or plug 29 into the channel or cam pathway 48 (if the helical configuration of the channel is wound in the opposite direction, the elongated member would be rotated counterclockwise to thread the connecting projection). When the connecting projection, cam follower or plug 29 is threaded in a helically shaped channel or cam pathway 48, the elongated member 24 moves distally within the cavity 46 of the headpiece 36. The elongated member is rotated until the connecting projection, cam follower or plug 29 contacts end wall 54 of the channel or cam pathway 48 and is thus set into a closed condition.

The proximal end portion 38 of the headpiece 36 includes an arrangement for positively engaging the pusher, typically a headpiece thereof. By such an engagement, the embolic device headpiece and the pusher headpiece will not rotate circumferentially in a manner independent of each other.

A preferred engagement arrangement in this regard includes an engagement member 56 of the headpiece 36 which mates with a corresponding engagement member 58 of a pusher headpiece 19. As shown in Figs. 3, 3a, 4 and 4a, the illustrated respective engagement members 56, 58 each embody a partial circumferential projection in the axial direction, and such projections contact one another and can be complementary with each other. When desired, the projections combine to form a circumference with engaging surfaces that contact one another. In a preferred embodiment, the projections combine to form a shape having an axial or central axis, such as a cylinder.

The engagement surfaces 62, 62a and 64, 64a can be along an axis aligned parallel to the central axis of the cylinder formed by the mated engagement members 56, 58. The engaging surfaces also need not be parallel to the central axis but can be at an acute angle to the central axis of the cylinder. Also, each engagement surface can be along a common plane that is parallel to the central axis of the cylinder. Alternatively, the engagement surfaces of each headpiece can be along separate planes that do not intersect. For example, each engagement surface could be along a different plane wherein each plane is separated by a distance. This would also include engagement surfaces of the same headpiece that are bevelled in the same direction at the same angle.

In yet another alternative, the engagement surfaces can be along separate planes that intersect. For example, in the illustrated embodiment the engagement surfaces 62, 62a of the headpiece 36 could be bevelled inwardly toward each other, or the engagement surfaces could be bevelled outwardly away from each other. The engagement surfaces typically can be planar, or flat, but can have a curved configuration or component. For example, the engagement surfaces could have a tongue and groove mating configuration wherein an engagement surface of one headpiece could have a tongue, and the corresponding engagement surface of the other headpiece could have a corresponding groove which mates with the tongue when the engagement members are engaged.

The engagement surfaces interact with each other to provide interference with independent circumferential movement of the headpieces while allowing independent movement of the embolic device 30 and the pusher 16 axially when it is desired to deploy the embolic device. The illustrated engagement member 56 of the embolic device headpiece 36 is a semi-circular projection 60 which includes engagement surfaces that are flats 62, 62a located on either side or edge of the projection 60. The flats 62, 62a in this illustrated embodiment engage corresponding engagement surfaces, such as flats 64, 64a, located on a semi-circular projection 66 of engagement member 58 of the pusher headpiece 19 shown in Figs. 4 and 4a.

As will be explained in more detail below, the arrangement for positively engaging the respective headpieces 19 and 36 functions as follows according to the illustrated preferred embodiment. The pusher headpiece 19 and the embolic device headpiece 36 engage each other to either resist or counteract torque applied to the proximal end portion 26 of the elongated member 24. Alternatively, the engagement of pusher headpiece 19 and embolic headpiece 36 can be used to rotate embolic device 30 by rotating pusher 16.

As illustrated in Figs. 4 and 4a, the illustrated pusher headpiece 19 also includes a proximal end portion 68. The proximal end portion 68 includes a joining element 70, which is illustratively shown as a tubular projection, for joining the headpiece 19 to the delivery tube 17. The distal end portion 20 of the delivery tube 17 engages, such as by fitting over, the joining member 70. The joining member 70 and the delivery tube 17 can be connected by weld, solder, adhesive or any other suitable method. The headpiece 19 also includes a passageway 72 which allows the elongated member 24 to extend through it and project from headpiece 19 of the pusher 16.

To secure the embolic device in the vascular occlusion deployment system, the embolic device 30 is preferably attached to the elongated member 24 of the pusher 16 by contacting the engagement member 56 of the embolic device 30 with engagement member 58 of the pusher 16 so that the flats or engagement surfaces 62, 62a and 64, 64a respectively of the respective engagement members 56 and 58 mate with each other. The elongated member 24 is then advanced distally within the pusher 16, and the connecting projection, cam follower or plug 29 is aligned with the notch 52 of the channel or cam pathway 48. The elongated member 24 is rotated in a direction that moves the connecting projection, cam follower or plug 29 within the channel or cam pathway 48. The elongated member 24 is rotated until the connecting projection, cam follower or plug 29 contacts the end wall 54 of the channel or cam pathway 48.

An alternative method of connecting the embolic device 30 to the elongate member 24 would be to position the elongated member 24 so that it extends out of the headpiece 19 of the pusher 16. The distal end portion 28 of the elongated member 24 is then positioned so that the connecting projection, cam follower or plug 29 is located at the notch 52 of the channel or cam pathway 48. The embolic device 30 is maintained in a substantially stationary position, such as by grasping by hand or by some other mechanism, and the elongated member 24 is rotated to move the connecting projection, cam follower or plug 29 into the channel or cam pathway 48. In lieu of rotating the elongated member 24, the elongated member 24 can be maintained in a stationary position, and the embolic device 30 can be rotated by hand or some other method to position the connecting projection, cam follower or plug 29 into the channel or cam pathway 48.

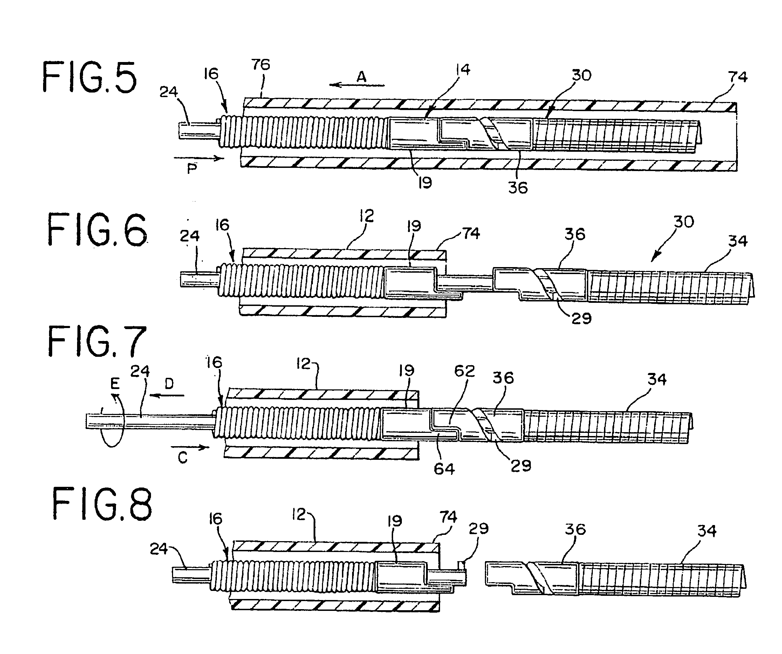

After the embolic device 30 has been attached to the elongated member 24, referring to Figs. 5 to 8, the delivery catheter 12 can be inserted into the vasculature system of a patient, and the distal end portion 74 of the catheter 12 can be positioned at a preselected location within a blood vessel, typically in conjunction with other devices and professional procedures as generally known in the art. The delivery unit 14 is inserted into a proximal end portion 76 of the catheter 12, and preferably the delivery unit 14 is advanced through the delivery catheter 12 until the embolic device 30 reaches the distal end portion 74 of the delivery catheter 12. If desired, the pusher headpiece 19 and the embolic device headpiece 36 can be engaged to increase column strength during the advancement of the pusher 16.

Once the embolic device 30 reaches the distal end portion 74 of the delivery catheter 12, the embolic device 30 may be moved out of the distal end portion 74 of the delivery catheter 12 in one of several ways. The delivery catheter 12 may be moved in a retrograde manner as indicated by arrow A. Alternatively, the pusher 16 may be advanced as indicated by arrow B. As a further alternative, the embolic device 30 may be advanced out of the delivery catheter 12 by advancing the elongated member 24 in a distal direction. Yet another alternative can be to use any of the above methods in conjunction with one another.

The embolic device 30 preferably includes a radiopaque marker so that the position of the embolic device 30 can be monitored by fluoroscopy. Referring to Fig. 6, after the embolic device 30 has exited the delivery catheter 12, if required, the elongated member 24 can be manipulated to more precisely place the embolic device 30 at the desired location. If it is determined that the embolic device 30 is in the wrong position and/or a different embolic device is required, the pusher 16 and the elongated member 24 can be retracted to move the embolic device 30 back into the delivery catheter 12. Once in the delivery catheter 12, the embolic device 30 can be repositioned or completely removed from the patient.

After it has been determined that the embolic device 30 is at the desired location within the patient, and if not already in engagement, the headpieces are so engaged. Typically, the pusher headpiece 19 is engaged with the embolic device headpiece 36 so that the corresponding flats 62, 62a and 64, 64a engage each other, as illustrated in Fig. 7. Engagement of the headpieces 19 and 36 can be accomplished as needed by advancing the pusher 16 in a distal direction as indicated by arrow C. It is also contemplated that in certain situations, it may be advantageous to engage the headpieces 19 and 36 by moving the elongated member 24 in a proximal direction as indicated by arrow D.

After the headpieces 19 and 36 have been engaged according to this illustrated embodiment, the embolic device 30 can be released by rotating the elongated member 24 so that the connecting projection, cam follower or plug 29 disengages and clears the channel or cam pathway 48. Preferably, the connecting projection, cam follower or plug 29 is unthreaded from the channel or cam pathway 48 by rotating the elongated member 24 circumferentially to provide torsional force to the elongated member, as illustrated by arrow E (or in the opposite circumferential direction if the channel or cam pathway is helically wound in the opposite direction). The torque applied to the elongated member 24 is resisted by the delivery tube 17 and headpiece 19. Additionally, the engagement along the headpieces 36 and 19, such as between the flats 62, 62a and 64, 64a respectively, limits or reduces rotational movement of the headpiece 36 of the embolic device 30 which causes the connecting projection, cam follower or plug 29 to unthread from channel or cam pathway 48 of the substantially stationary headpiece 36. The headpieces 36 and 19 maintain contact during release of the embolic device 30. The engagement between the headpieces 36 and 19 also limits or reduces any undesired rotational movement of the embolic device 30. When the pusher 16 is comprised of a coiled wire, it is preferable that the channel or cam pathway 48 of the embolic headpiece 36 be configured so that the elongated member 24 is rotated in a direction opposite of the wind of the coil during unthreading to avoid buckling or kinking the pusher 16.

It is also contemplated that there may be situations where it would be advantageous to disengage the connecting projection, cam follower or plug 29 from the channel or cam pathway 48 by rotating the embolic device 30 while maintaining the elongated member 24 in a substantially stationary position. By this approach, the pusher 16 is rotated and the rotational movement of the pusher 16 is translated along it, through the headpieces, to the embolic device. At the same time, the elongated member is maintained in a substantially stationary position, resulting in the unthreading in the connecting projection, cam follower or plug 29 from the channel or cam pathway 48.

As illustrated in Fig. 8, after the connecting projection, cam follower or plug 29 has been rotated and moved so as to clear the channel or cam pathway 48, the embolic device 30 can be released for deployment at a desired location within the patient such as within or at an aneurysm. The pusher 16 can now be retracted through the delivery catheter 12 and removed from the patient.

## Claims

1. An embolic device delivery system (10), comprising:
a pusher (16) having a proximal end portion and a distal end portion; ,
an elongated member (24) having a proximal end portion (26) and a distal end portion (26), said elongated member positioned along the pusher (16), said elongated member and said pusher being relatively rotatable;
a connecting projection (29) extending in a generally radial direction from the distal end portion of the elongated member (24), said connecting projection being in operative camming communication with a pathway of a proximal portion of an embolic device to removably secure the embolic device to the elongated member; and
said distal end portion of the pusher contacting the embolic device (30) to essentially prevent rotational movement of the embolic device with respect to the pusher.

2. The delivery system of claim 1, wherein the pathway of the embolic device (30) comprises a generally helical configuration.

3. The delivery system of claim 1, wherein the distal end portion of the pusher (16), includes an engagement member (58) and the embolic device (30) includes an engagement member (56), and wherein the contacting of the distal end portion of the pusher and the embolic device to essentially prevent rotational movement of the embolic device comprises mating the engagement member of the pusher with the engagement member of the embolic device.

4. The delivery system of claim 3, wherein:
the engagement member (58) of the pusher (16) and the engagement member (56) of the embolic device (30) mate to form a shape having an axial axis;
the engagement member of the pusher including at least one engagement surface that is axially oriented and extends in a direction that is generally parallel to the axial axis;
the engagement member of the embolic device including at least one engagement surface (68) that is axially oriented and extends in a direction that is generally parallel to the axial axis; and
the at least one engagement surface (64) of the engagement member of the pusher contacting the at least one engagement surface of the engagement member of the embolic device when said engagement members mate.

5. The delivery system of claim 4, wherein the at least one engagement surface of the engagement member (58) of the pusher (16) and the at least one engagement surface (62) of the engagement member (56) of the embolic device (30) are substantially planar.

6. The delivery system of claim 3, wherein the pusher comprises a delivery tube (17) and a pusher headpiece (19), and the engagement member (58) of the pusher is located on the pusher (16) headpiece.

7. The delivery system of claim 3, wherein the embolic device (30) comprises an embolic element (34) and an embolic device headpiece (36), and the engagement member (56) of the embolic device is located on the embolic device headpiece.

8. The delivery system of claim 7, wherein the pathway is a threadable channel (48) of the embolic device headpiece (36).

9. A kit which comprises an embolic device delivery system as claimed in claim 1, and an embolic device (30) which has a proximal end portion and a distal end portion, with a pathway provided in the proximal end portion, in which:
the elongated member (24) includes a distal end portion which has a connecting projection (29) extending in a generally radial direction from the distal end portion of the elongated member, said connecting projection engaging the pathway to removably secure the embolic device to the elongated member; and
the distal end portion of the pusher (16) contacting the proximal end portion of the embolic device substantially to prevent rotation of the embolic device with respect to the pusher.

## Patentansprüche

1. Abgabesystem (10) mit einer embolischen Einheit, das aufweist:
einen Schieber (16), der einen proximalen Endbereich und einen distalen Endbereich hat;
ein längliches Element (24) der einen proximalen Endbereich (26) und einen distalen Endbereich (28) hat, wobei das längliche Element dem Schieber (16) entlang angeordnet ist, wobei das längliche Element und der Schieber relativ zueinander drehbar sind;
einen verbindenden Vorsprung (29), der sich in einer im Allgemeinen radialen Richtung von dem distalen Endbereich des länglichen Elementes (24) erstreckt, wobei der verbindende Vorsprung in betrieblich führendem Eingriff in einen Weg eines proximalen Bereiches einer embolischen Einheit ist, um die embolische Einheit entfernbar an dem länglichen Element zu sichern; und
wobei der distale Endbereich des Schiebers die embolische Einheit (30) berührt, um die Drehbewegung der embolischen Einheit in Bezug auf den Schieber im Wesentlichen zu verhindern.

2. Abgabesystem nach Anspruch 1, bei dem der Weg der embolischen Einheit (30) eine im Allgemeinen wendelartige Gestaltung hat.

3. Abgabesystem nach Anspruch 1, bei dem der distale Endbereich des Schiebers (16) ein Eingriffselement (58) umfasst und die embolische Einheit (30) ein Eingriffselement (56) umfasst und bei dem das Berühren des distalen Endbereiches des Schiebers und der embolischen Einheit, um die Drehbewegung der embolischen Einheit im Wesentlichen zu verhindern, das Zusammenbringen des Eingriffselements des Schiebers mit dem Eingriffselement der embolischen Einheit aufweist.

4. Abgabesystem nach Anspruch 3, bei dem
das Eingriffselement (58) des Schiebers (16) und das Eingriffselement (56) der embolischen Einheit (30) einander entsprechen, um eine Form mit einer axialen Achse zu bilden;
wobei das Eingriffselement des Schiebers wenigstens eine Anlagefläche umfasst, die axial ausgerichtet ist und sich in eine Richtung erstreckt, die im Allgemeinen parallel zu der axialen Achse ist;
wobei das Eingriffselement der embolische Einheit wenigstens eine Anlagefläche (62) umfasst, die axial ausgerichtet ist und sich in eine Richtung erstreckt, die im Allgemeinen parallel zu der axialen Achse ist; und
wobei die wenigstens eine Anlagefläche (64) des Eingriffselementes des Schiebers die wenigstens eine Anlagefläche des Eingriffselementes der embolischen Einheit berührt, wenn die Eingriffselemente aneinander gepasst sind.

5. Abgabesystem nach Anspruch 4, bei dem die wenigstens eine Anlagefläche des Eingriffselementes (58) des Schiebers (16) und die wenigstens eine Anlagefläche (62) des Eingriffselementes (16) der embolischen Einheit (30) im Wesentlichen eben sind.

6. Abgabesystem nach Anspruch 3, bei dem der Schieber ein Abgaberohr (17) und ein Schieberkopfstück (19) aufweist und sich das Eingriffselement (58) des Schiebers auf dem Kopfstück des Schiebers (16) befindet.

7. Abgabesystem nach Anspruch 3, bei dem die embolische Einheit (30) ein embolisches Element (34) und ein Kopfstück (36) der embolischen Einheit aufweist und das Eingriffselement (56) der embolische Einheit sich auf dem Kopfstück der embolischen Einheit befindet.

8. Abgabesystem nach Anspruch 7, bei dem der Weg ein schraubenartiger Kanal (48) des Kopfstücks (36) der embolischen Einheit ist.

9. Basisausrüstung, welche ein Abgabesystem für eine embolische Einheit nach Anspruch 1, und eine embolische Einheit (30), die einen proximalen Endbereich und einen distalen Endbereich hat, wobei ein Weg in dem proximalen Endbereich vorgesehen ist, aufweist, wobei:
das längliche Element (24) einen distalen Endbereich umfasst, der einen verbindenden Vorsprung (29) hat, welcher sich in einer im Allgemeinen radialen Richtung von dem distalen Endbereich des länglichen Elementes erstreckt, wobei der verbindende Vorsprung in den Weg eingreift, um die embolische Einheit lösbar an dem länglichen Elemente zu sichern; und
wobei der distale Endbereich des Schiebers (16) den proximalen Endbereich der embolischen Einheit berührt, um die Drehung der embolischen Einheit in Bezug auf den Schieber im Wesentlichen zu verhindern.

## Revendications

1. Système de pose (10) de dispositif embolique, comprenant :
■ un poussoir (16) ayant une partie d'extrémité proximale et une partie d'extrémité distale ;
■ un élément allongé (24) ayant une partie d'extrémité proximale (26) et une partie d'extrémité distale (28), ledit élément allongé étant positionné le long du poussoir (16), ledit élément allongé et ledit poussoir étant relativement rotatifs ;
■ une saillie de raccordement (29) s'étendant dans une direction généralement radiale à partir de la partie d'extrémité distale de l'élément allongé (24), ladite saillie de raccordement étant en communication de mise en prise opérationnelle avec une voie de passage d'une partie proximale d'un dispositif embolique pour fixer de manière amovible le dispositif embolique à l'élément allongé ; et
■ ladite partie d'extrémité distale du poussoir étant en contact avec le dispositif embolique (30) pour empêcher essentiellement le mouvement de rotation du dispositif embolique par rapport au poussoir.

2. Système de pose selon la revendication 1, dans lequel la voie de passage du dispositif embolique (30) comprend une configuration généralement hélicoïdale.

3. Système de pose selon la revendication 1, dans lequel la partie d'extrémité distale du poussoir (16) comprend un élément de mise en prise (58) et le dispositif embolique (30) comprend un élément de mise en prise (56), et dans lequel le contact de la partie d'extrémité distale du dispositif de poussée et du dispositif embolique pour empêcher essentiellement le mouvement de rotation du dispositif embolique comprend le couplage de l'élément de mise en prise du poussoir avec l'élément de mise en prise du dispositif embolique.

4. Système de pose selon la revendication 3, dans lequel :
■ l'élément de mise en prise (58) du poussoir (16) et l'élément de mise en prise (56) du dispositif embolique (30) se couplent pour former une forme ayant un axe axial ;
■ l'élément de mise en prise du poussoir comprenant au moins une surface de mise en prise qui est orientée de manière axiale et s'étend dans une direction qui est généralement parallèle à l'axe axial ;
■ l'élément de mise en prise du dispositif embolique comprenant au moins une surface de mise en prise (62) qui est orientée de manière axiale et s'étend dans une direction qui est généralement parallèle à l'axe axial ; et
■ la au moins une surface de mise en prise (64) de l'élément de mise en prise du poussoir étant en contact avec la au moins une surface de mise en prise de l'élément de mise en prise du dispositif embolique lorsque lesdits éléments de mise en prise se couplent.

5. Système de pose selon la revendication 4, dans lequel la au moins une surface de mise en prise de l'élément de mise en prise (58) du poussoir (16) et la au moins une surface de mise en prise (62) de l'élément de mise en prise (56) du dispositif embolique (30) sont sensiblement planes.

6. Système de pose selon la revendication 3, dans lequel le poussoir comprend un tube de pose (17) et une pièce à main (19) de poussoir, et l'élément de mise en prise (58) du poussoir est positionné sur la pièce à main de poussoir (16).

7. Système de pose selon la revendication 3, dans lequel le dispositif embolique (30) comprend un élément embolique (34) et une pièce à main (36) de dispositif embolique, et l'élément de mise en prise (56) du dispositif embolique est positionnée sur la pièce à main de dispositif embolique.

8. Système de pose selon la revendication 7, dans lequel la voie de passage est un canal pouvant être fileté (48) de la pièce à main (36) de dispositif embolique.

9. Ensemble qui comprend un système de pose de dispositif embolique selon la revendication 1, et un dispositif embolique (30) qui a une partie d'extrémité proximale et une partie d'extrémité distale, avec une voie de passage prévue dans la partie d'extrémité proximale, dans lequel :
■ l'élément allongé (24) comprend une partie d'extrémité distale qui a une saillie de raccordement (29) s'étendant dans une direction généralement radiale à partir de la partie d'extrémité distale de l'élément allongé, ladite saillie de raccordement mettant en prise la voie de passage pour fixer de manière amovible le dispositif embolique sur l'élément allongé ; et
■ la partie d'extrémité distale du poussoir (16) étant en contact avec la partie d'extrémité proximale du dispositif embolique pour empêcher sensiblement la rotation du dispositif embolique par rapport au poussoir.
